(19) Europäisches Patentamt
European Patent Office
Office européen des brevets

(11) Veröffentlichungsnummer: **0 085 937**
**B1**

(12) # EUROPÄISCHE PATENTSCHRIFT

(45) Veröffentlichungstag der Patentschrift: **27.08.86**

(51) Int. Cl.⁴: **C 07 D 321/10,** C 11 B 9/00

(21) Anmeldenummer: **83100970.9**

(22) Anmeldetag: **02.02.83**

(54) **Riechstoff mit tricyclischer Ringstruktur.**

(30) Priorität: **10.02.82 DE 3204627**

(43) Veröffentlichungstag der Anmeldung: **17.08.83 Patentblatt 83/33**

(45) Bekanntmachung des Hinweises auf die Patenterteilung: **27.08.86 Patentblatt 86/35**

(84) Benannte Vertragsstaaten: **CH DE FR GB LI NL**

(56) Entgegenhaltungen: **EP-A-0 024 473**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Upadeck, Horst, Dr., Kempenweg 18 A, D-4006 Erkrath (DE)**
Erfinder: **Bruns, Klaus, Dr., Notburgaweg 6, D-4150 Krefeld/Traar (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 085 937 B1

ACTORUM AG

**Beschreibung**

Es wurde gefunden, dass 4,6-Dioxa-5-ethyl-methyltricyclo[7.2.1.$0^{2,8}$]dodec-1o-en der Formel

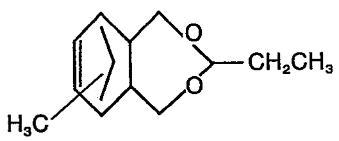

in Form des Isomerengemisches, in welchem $CH_3$ die Positionen 1, 9, 10, 11 oder 12 einnimmt, ein wertvoller neuer Riechstoff ist.

Die Herstellung der neuen Verbindung erfolgt nach an sich bekannten Syntheseverfahren der organischen Chemie. Man geht dabei von Methylcyclopentadien und 1,3-Dioxa-2-ethyl-5-cyclohepten aus und setzt diese in einer Diels-Alder-Reaktion bei 150–210 °C in etwa 5–10 Stunden, vorzugsweise bei ca. 200 °C in ca. 6 Stunden, im Autoklaven um. Nach destillativer Aufarbeitung fällt das Produkt als Gemisch von Struktur- und Stereoisomeren an und wird ohne weitere Trennung als Riechstoff eingesetzt.

In der DE-OS 2 918 168 ist die Herstellung von 4,6-Dioxa-5-ethyl-tricyclo[7.2.1.$0^{2,8}$]dodec-10-en durch Diels-Alder-Reaktion von Cyclopentadien mit 1,3-Dioxa-2-ethyl-5-cyclohepten beschrieben. Das dabei anfallende Isomerengemisch ist ein wertvoller Riechstoff, der eine frische Laub-, Humus-, Champignon-Note besitzt.

Demgegenüber zeichnet sich das beanspruchte Isomerengemisch durch eine ausgeprägte Sandelholznote mit einer an Datteln erinnernden fruchtigen Beinote aus. Sandelholzdüfte stellen in der Parfümindustrie sehr gefragte und kostspielige Komponenten in Riechstoff-Kompositionen dar. Die beanspruchten neuen Verbindungen können in dieser Richtung als preisgünstiges, leicht zugängliches Material verwendet werden.

In Verbindung mit anderen Riechstoffen und gegebenenfalls gängigen Parfümbestandteilen lassen sich die neuen Verbindungen zu neuen, interessanten Riechstoffkompositionen kombinieren. Dabei werden etwa 1–50 Gew.-% der neuen Verbindungen, bezogen auf die gesamte Komposition, eingesetzt. Derartige Kompositionen können zur Parfümierung von Kosmetika, wie Duftwässern, Cremes, Lotionen, Aerosolen, Toilettenseifen, in der Extrait-Parfümerie, sowie zur Geruchsverbesserung von technischen Artikeln, wie Wasch- und Reinigungsmitteln, Desinfektionsmitteln, Textilbehandlungsmitteln u.dgl. dienen. Zur Parfümierung der verschiedenen Produkte werden diesen die Kompositionen in Mengen von 0,05–2 Gew.-%, bezogen auf das gesamte Produkt, zugesetzt.

Beispiel 1

Herstellung von 4,6-Dioxa-5-ethyl-methyltricyclo[7.2.1.$0^{2,8}$]dodec-10-en

64,1 g (0,5 Mol) 1,3-Dioxa-2-ethyl-5-cyclohepten, das in üblicher Weise durch Acetalisierung von Propionaldehyd mit cis-2-Buten-1,4-diol gewonnen wurde, und 48,1 g (0,3 Mol) Methylcyclopentadien-Dimer wurden 6 h bei 200 °C im Autoklaven erhitzt. Das Rohprodukt wurde im Vakuum fraktioniert destilliert. Es wurden 42,4 g (40.7% d.Th.) an 4,6-Dioxa-5-ethyl-methyltricyclo[7.2.1.$0^{2,8}$]dodec-10-en bei 98–102 °C/3.3 mbar erhalten.

$n^{20}_D = 1,4914$

Geruch: Sandelholz, Dattel, fruchtig

Beispiel 2

Holziger Phantasie-Chypre-Komplex

| | |
|---|---|
| 4,6-Dioxa-5-ethyl-methyltricyclo-[7.2.1.$0^{2,8}$]dodec-10-en | 200 Gew.-Teile |
| Vetiverylacetat | 200 Gew.-Teile |
| Linalylacetat | 200 Gew.-Teile |
| Hydroxycitronellal | 120 Gew.-Teile |
| γ-Methyljonon | 120 Gew.-Teile |
| 1-Citronellol | 80 Gew.-Teile |
| Aldehyd C 14 (γ-Undecalacton) (10% in DEP) | 40 Gew.-Teile |
| Undecylenaldehyd (10% in DEP) | 40 Gew.-Teile |
| | 1000 Gew.-Teile |

(DEP = Diethylphthalat)

**Patentansprüche**

1. 4,6-Dioxa-5-ethyl-methyltricyclo[7.2.1.$0^{2,8}$]dodec-10-en der Formel

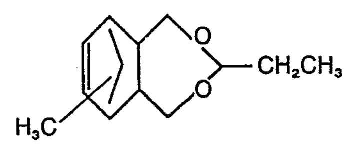

in Form des Isomerengemisches, in welchem $CH_3$ die Positionen 1, 9, 10, 11 oder 12 einnimmt.

2. Herstellung von 4,6-Dioxa-5-ethyl-methyltricyclo[7.2.1.$0^{2,8}$]dodec-10-en gemäss Anspruch 1 durch Umsetzung von Methylcyclopentadien mit 1,3-Dioxa-2-ethyl-5-cyclohepten bei 150–210 °C im Autoklaven.

3. Verwendung von 4,6-Dioxa-5-ethyl-methyltricyclo[7.2.1.$0^{2,8}$]dodec-10-en in Form des Isomerengemisches gemäss Anspruch 1 als Riechstoff.

4. Riechstoffkompositionen, gekennzeichnet durch einen Gehalt an 4,6-Dioxa-5-ethyl-methyltricyclo[7.2.1.$0^{2,8}$]dodec-10-en in Form des Isomerengemisches gemäss Anspruch 1.

**Claims**

1. 4,6-dioxa-5-ethylmethyltricyclo[7.2.1.$0^{2,8}$]dodec-10-ene corresponding to the following formula

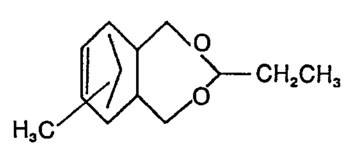

in the form of the isomer mixture, in which $CH_3$ occupies the 1, 9, 10, 11 or 12 position.

2. Preparation of 4,6-dioxa-5-ethylmethyltricyclo[7.2.1.$0^{2,8}$]dodec-10-ene according to Claim 1 by reaction of methyl-cyclopentadiene with 1,3-dioxa-2-ethyl-5-cycloheptene in an autoclave at 150 to 210 °C.

3. The use of 4,6-dioxa-5-ethylmethyltricyclo[7.2.1.$0^{2,8}$]dodec-10-ene in the form of the isomer mixture according to Claim 1 as a perfume.

4. Perfume compositions, characterized by a content of 4,6-dioxa-5-ethylmethyltricyclo-[7.2.1.$0^{2,8}$]dodec-10-ene in the form of the isomer mixture according to Claim 1.

## Revendications

1. 4,6-dioxa-5-éthyl-méthyltricyclo[7.2.1.$0^{2,8}$]dodéc-10-ène répondant à la formule générale

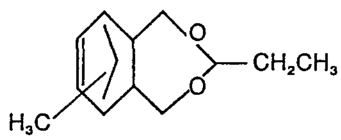

sous forme d'un mélange d'isomères, dans lequel $CH_3$ peut prendre les positions 1, 9, 10, 11, ou 12.

2. Fabrication du 4,6-dioxa-5-éthyl-méthyltricyclo[7.2.1.$0^{2,8}$]dodéc-10-ène, suivant la revendication 1, par réaction de méthylcyclopentadiène avec 1,3-dioxa-2-éthyl-5-cycloheptène, à 150 à 210 °C, dans un autoclave.

3. Utilisation du 4,6-dioxa-5-éthyl-méthyltricyclo[7.2.1.$0^{2,8}$]dodéc-10-ène sous la forme du mélange d'isomères suivant la revendication 1, comme matière odorante.

4. Compositions pour parfums, caractérisées en ce qu'elles contiennent une certaine proportion de 4,6-dioxa-5-éthyl-méthyltricyclo[7.2.1.$0^{2,8}$]dodéc-10-ène, sous la forme du mélange d'isomères suivant la revendication 1.